# EUROPEAN PATENT APPLICATION

(11) **EP 1 251 171 A1**
(43) Date of publication of application: **23.10.2002**
(21) Application number: 00978059.4
(22) Date of filing: 01.12.2000
(51) Int. Cl.: C12N 15/12, C07K 14/47, A61K 38/16, A61K 31/711, A61K 48/00, A61P 11/00

(54) **NOVEL ENDOPLASMIC RETICULUM-LOCALIZED PROTEIN, REMEDIES CONTAINING THE SAME FOR DISEASES ASSOCIATED WITH GLYCOPROTEIN ABNORMALITY, DNA ENCODING THIS PROTEIN AND GENE REMEDIES CONTAINING THE SAME FOR TREATING DISEASES ASSOCIATED WITH GLYCOPROTEIN ABNORMALITY**

(30) Priority: 03.12.1999 JP 34479599
(71) Applicant: HISAMITSU PHARMACEUTICAL CO. INC., Tosu-shi, Saga-ken 841-0017 (JP); Kai, Hirohumi, Kumamoto-shi, Kumamoto 862-0949 (JP)
(72) Inventor: KAI, Hirohumi, Kumamoto-shi, Kumamoto 862-0949 (JP); YOSHITAKE, Kazuhisa, Hisamitsu Pharmaceut.Co.,Inc., Tsukuba-shi, Ibaraki 305-0856 (JP)
(74) Representative: Grünecker, Kinkeldey, Stockmair & Schwanhäusser Anwaltssozietät
(86) International application number: JP0008529
(87) International publication number: WO01040463

(57) **Abstract**

A protein comprising the amino acid sequence set forth in SEQ ID NO:1 in the Sequence Listing and a mutant thereof, possessing the glycoprotein production regulating activity, DNA comprising the nucleotide sequence set forth in SEQ ID NO:2 in the Sequence Listing and a mutant thereof, as well as a therapeutic agent using any of the foregoing for the treatment of glycoprotein aberration disorders (e.g., cystic fibrosis), including gene therapy.

## Description

### Technical Field

This invention relates to novel endoplasmic localization proteins (which will be referred to as "ELP" or "ELP protein(s)" hereafter) possessing the glycoprotein production regulating activity and genes encoding them.

### Background Art

Endoplasmic reticulum (also referred to as "ER" hereafter) is an important organelle that provides a field for the folding or the assembly of newly produced secretory or membrane-binding glycoproteins. Calnexin, calreticulin and the like have been hitherto known as the proteins responsible for the function of folding or assembling newly produced glycoproteins in endoplasmic reticulum.

Calnexin is a Ca²⁺ binding I type membrane protein present in rough endoplasmic reticulum. It is characterized by having a molecular weight of 65 kDa (573 amino acids) deduced from its nucleotide sequence, by having a large part of its N-terminus which contains a substrate binding domain oriented towards lumenal ER, and by having a cytoplasmic domain with 89 amino acid at its C-terminus, the cytoplasmic domain containing a phosphorylation site and an ER localization signal (RKPRRE). See Wada, I et al., J. Bio. Chem., 266, 29, 19599-19610, 1991.

Calreticulin is also a soluble Ca²⁺ binding protein present in the ER and is a soluble homolog of calnexin characterized by having a molecular weight of 46 kDa (400 amino acids) and an ER localization signal (KDEL) at its C-terminus. See Michalak, M. et al., Exp. Cell Res., 197, 91-99, 1991.

It has been shown that calnexin and calreticulin recognize a monoglycosylated sugar chain of sugar chain of the asparagine bonding type for a glycoprotein and cause the protein to be retained in the ER by binding to its folding intermediate, but that they do not bind with maturation proteins. These proteins function as molecular chaperones in the ER.

The reports thus far have shown that calnexin and calreticulin are involved in the control of folding or assembly of various secretory or membrane-binding glycoproteins such as those mentioned below, as well as possess the function of suppressing the expression and the secretion of aberrant proteins having undergone folding abnormalities. They are cystic fibrosis transmembrane conductance regulator (referred to as "CFTR" hereafter), nicotine-agonistic acetylcoline receptor, α 1-anti-trypsin, α 1-anti-chimotrypsin, α-fetoprotein, complement 3 (C3), apo β-100, transferrin, LDL receptor, MHC class I, MHC class II, influenza hemaglutinin, T-cell receptor, integrin, glycosetransporter (Glut 1), and the like.

Thus, calnexin and calreticulin are sugar chain associated functional molecules the physiological significance of which has gaining the wide recognition of their importance, because they are involved in the quality control at the initial stage of glycoprotein synthesis. Consequently, the discovery of an endogenous substance that regulates the functions of calnexin and calreticulin can be epoch-making therapy for treating glycoprotein aberration disorders caused by mutant glycoproteins undissociated from calnexin or calreticulin and hence their long resident time in the ER.

Specifically, the deficiency of the 508th amino acid, i.e., phenylalanine, of CFTR ( Δ F508 CFTR) expressed in endothelial cells is found in about 70% of cystic fibrosis patients. In such cases Δ F508 CFTR cannot be dissociated from calnexin because of its folding abnormality and has been retained in the ER for a prolonged period of time; it is finally decomposed, thus resulting in no expression of CFTR on cell membrane. However, when Δ F508 CFTR-expressing cells are grown under the temperature condition of 30 ° c or less to suppress the function of calnexin, the expression of Δ F508 CFTR is observed on the cell membrane. With the addition of DMSO which is a chemical chaperon, a similar phenomenon is also noted. These facts suggest that the lowering of the function for calnexin or calreticulin is effective for the treatment of glycoprotein aberration disorders.

### Disclosure of the Invention

This invention was made in view of the aforementioned circumstances and it aims at providing novel proteins possessing glycoprotein production regulating activity and genes encoding the proteins.

As a result of having pursued diligent investigations, the present inventors found proteins possessing the glycoprotein production regulating activity which are different from calnexin or calreticulin and genes encoding them.

Specifically, this invention provides the proteins described in 1-5 below and DNAs:
1. A protein comprising the amino acid sequence set forth in SEQ ID NO:1 in the Sequence Listing.
2. A protein comprising an amino acid sequence derivable from the substitution or the deletion of one or more amino acids in the amino acid sequence SEQ ID NO:1 in the Sequence Listing, or from the addition of one or more amino acids to the amino acid sequence set forth in SEQ ID NO:1 in the Sequence Listing, the protein possessing glycoprotein production regulating activity.
3. DNA comprising the nucleotide sequence set forth in SEQ NO:2 in the Sequence Listing.
4. DNA comprising a nucleotide sequence derivable from the substitution or the deletion of one or more nucleotides in the nucleotide sequence set forth in SEQ ID NO:2 in the Sequence Listing, or from the addition of one or more nucleotides to the nucleotide sequence set forth in SEQ ID NO:2 in the Sequence Listing.
5. Antisense DNA to the DNA described in 3 or 4 above.

This invention also provides a therapeutic agent for the treatment of a glycoprotein aberration disorder, comprising a protein described in 1 or 2 above. Here, the protein may be in the form of a pharmaceutically acceptable salt thereof.

Further, this invention provides a gene therapy agent for the treatment of a glycoprotein aberration disorder, comprising DNA described in 3 or 4 above.

In the specification, unless stated otherwise, DNA (including cDNA) refers to that comprising double strands, a sense strand and an antisense strand; and RNA refers to that comprising a single strand and antisense DNA refers to that comprising a single strand.

### Brief Description of the Drawings

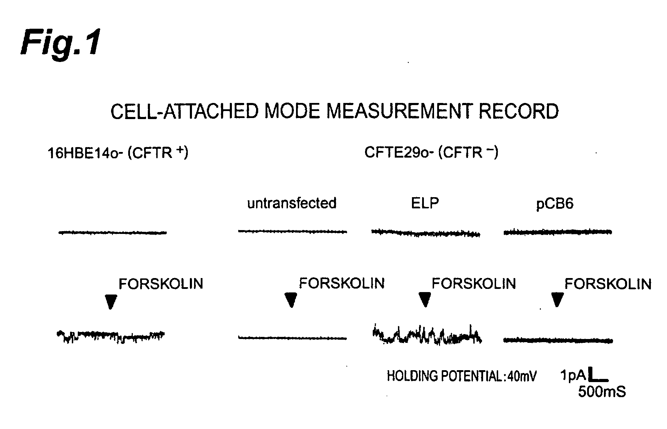

Fig. 1 is a representation showing the current waveforms resulting from the examination of the CFTR expression capabilities of 16HBE14o⁻ cells expressing the normal CFTR and various kinds of CFTE29o⁻ cells including those into which the ELP gene has been introduced in accordance with the patch clamp method.

### Best Mode for Carrying Out the Invention

This invention will be described in detail hereafter.

### (ELP Proteins and ELP Genes)

The present inventors performed PCR by employing as a template cDNA from a human cancer cell line and oligonucleotide primers corresponding to the 5'-end and the 3'-end for the purpose of cloning the full-length of calnexin and separated its products by agarose gel electrophoresis. Subsequently, the cDNA was subjected to cloning, where an Original TA Cloning Kit was used to clone it into a PCR2.1 vector according to the conventional method; and the nucleotide sequence of the resultant DNA was determined. The amino acid sequence corresponding to the nucleotide sequence was further determined. The protein comprising this amino acid sequence can be produced by incorporating the DNA into a suitable vector and causing it to express. As predicted, the resulting protein is comprised of 256 amino acids and its apparent molecular weight is about 38 kDa.

The former nucleotide sequence is designated SEQ ID NO:2 and the latter amino acid sequence is designated SEQ ID NO:1: they are shown in the Sequence Listing, respectively. The protein comprising the amino acid sequence set forth in SEQ ID NO:1 is referred to as "the ELP of this invention" and DNA comprising the nucleotide sequence set forth in SEQ ID NO:2 is referred to as "the DNA of this invention."

The physiological activity of the ELP of this invention was examined in the manner described below.

When the expression of ELP in a normal cell was first suppressed by treatment with antisense DNA to the ELP, its effect on the expression of CFTR on cell membrane was investigated. Consequently, the CFTR expression on the cell membrane was reduced. Further, the effect on the CFTR expression on the cell membrane was investigated when the ELP gene (i.e., the DNA of this invention) was introduced into a normal cell and was allowed to be expressed excessively. Consequently, the CFTR expression on the cell membrane was markedly enhanced. These experimental results demonstrated that the ELP of this invention had the function of regulating the production of the glycoprotein.

When the ELP gene was introduced into a human tracheal endothelial cell expressing the Δ 508CFTR gene which in turn could not express the CFTR that had the normal function on cell membrane and was allowed to be expressed excessively, its effect on the expression of CFTR on the cell membrane was investigated. Consequently, the CFTR expression on the cell membrane was markedly enhanced, and in addition, the expressed CFTR had the normal function. This result demonstrated that the ELP of this invention had the function of restoring the lowered production of a glycoprotein which was caused by mutant glycoproteins undissociated from calnexin which were newly produced because of gene mutation and hence their long resident time in ER.

Accordingly, the ELP of this invention and the DNA of this invention will be useful in the treatment of glycoprotein aberration disorders caused by mutant glycoproteins undissociated from calnexin and hence their long resident time in the ER, including cystic fibrosis, juvenile pulmonary emphysema, Tay-Sacks disease, congenital sucrase isomaltase deficiency, and familial hypercholesterolaemia.

Among the polypeptides derivable from the substitution or the deletion of one or more amino acids from the amino acid sequence set forth in SEQ ID NO:1, or from the addition of one or more amino acids to the amino acid sequence set forth in SEQ ID NO:1, there are those possessing the glycoprotein production regulating activity. Such polypeptides are mutant (or variant) proteins of the ELP of this invention and are encompassed by the ELPs of this invention insofar as they possess the glycoprotein production regulating activity. Likewise, the polynucleotides derivable from the substitution or the deletion of one or more nucleotides from the nucleotide sequence set forth in SEQ ID NO:2, or from the addition of one or more nucleotides to the nucleotide sequence set forth in SEQ ID NO: 2 are encompassed by the DNAs of this invention insofar as they encode the ELP mutant proteins of this invention.

Sugar chains have been added to a large number of ordinary proteins and the sugar chain addition can be adjusted by the conversion of one or more amino acids. The polypeptides the sugar chain addition of which has been adjusted in the amino acid sequence set forth in SEQ ID NO:1 are encompassed by the ELPs of this invention insofar as they possess the glycoprotein production regulating activity. The polynucleotides encoding the aforementioned polypeptides are also encompassed by the DNAs of this invention.

### (Gene Therapy)

The DNA of this invention can be incorporated into a therapeutic vector and can be used in the gene therapy for various disorders recited previously.

Attempts have been made on many of the glycoprotein aberrant disorders thus far. For example, an Adenovirus vector is mainly used as the therapeutic vector in the gene therapy of cystic fibrosis. Although it can theoretically incorporate a gene that is as large as about 7 kb, the normal CFTR gene is itself 6.5 kb and the size will be larger than that after the incorporation of a promoter, which will then necessitate the incorporation of a very large therapeutic gene. A large number of gene therapy cases have been conducted that utilize Adenovirus vectors having the incorporation of such a large therapeutic gene (CFTR). In no case sufficient therapeutic effects have been obtained, which addresses the current status. This is because 50-80% of CFTR molecules turns out to be decomposed without being expressed on the cell membrane even if the normal CFTR gene has been introduced into the cell. Consequently, these facts need to be considered in order for an effective amount of the CFTR to be expressed. An Adeno-associated virus vector, being less toxic, is expected to be the next generation of the viral vectors. However, it has the limitation such that the size of the therapeutic gene capable of being inserted is 4.5 kb or less.

Therefore, the smaller the size of the therapeutic gene for use in the gene therapy is, the greater the generality and utility becomes. As compared with a conventional method where the CFRT gene is directly used, the DNA of this invention is as small as 0.8 kb and thus, it can be said to be highly useful as a therapeutic gene.

The vectors with which the DNA of this invention is used are not particularly limited; and for example, there may be used vectors derived from recombinant vaccine viruses, polio virus, influenza virus, Adenovirus, Adeno-associated virus, herpes simplex virus, HIV virus, Sendai virus, and the like. In addition, the sequences involved in transformation may be introduced into the aforementioned vectors that include a suitable promoter, origin of replication, selectable marker, RNA splicing site, and polyadenylation signal.

The DNA of this invention can be incorporated into the vector and can be used as a gene therapy agent by following a conventional technique. Specifically, when the gene therapy is to be conducted, the recombinant viral vector may be introduced into the targeted cell by being brought into contact with the cell targeted for therapy, or by being inserted into an expression vector such as plasmid vector. Gene transfer may be carried out by methods known in the art, including the calcium phosphate method, the liposome method, electroporation, and the DEAE-dextran method.

### (Antisense DNAs)

In this invention, antisense DNA to the DNA comprising the nucleotide sequence set forth in SEQ ID NO:2 is encompassed by the DNAs of this invention. Such antisense DNA has a complementary nucleotide sequence to the mRNA that should be intrinsically transcribed. By forming a base pair with the mRNA, it blocks the flow of genetic information, thus suppressing the expression of gene and inhibiting the synthesis of the final product, the ELP protein. The antisense DNA that can be used in this invention has a sequence that is complementary to at least part of the nucleotide sequence set forth in SEQ ID NO:2 and it encodes antisense RNA, which is an oligonucleotide capable of specifically hybridizing to the nucleotide sequence of the mRNA.

As used herein, the term "oligonucleotide(s)" means an oligonucleotide or an analog thereof formed from naturally occurring bases and the sugar moieties bonded thereto through original phosphodiester bonds. Thus, the first group members included in this term are naturally occurring species, naturally occurring subunits or synthetic species formed from homologs of the foregoing. Here, the "subunit" refers to a combination of bases and sugars bonded to the adjacent subunit through phosphodiester bonds or any other bonds. Although the second group members of oligonucleotides are the analogs of the aforementioned oligonucleotides and function similarly to the latter, they are provided with residues having non-naturally occurring moieties. Included by these are oligonucleotides wherein the phosphate groups, sugar moieties, the 3'-end or the 5'-end has been chemically modified for the purpose of increasing stability. Specifically, there are mentioned an oligophosphorothioate and an oligomethylphosphonate where one of the oxygen atoms for the phosphodiester group between nucleotides is replaced by sulfur and by -CH₃, respectively. The phophodiester bond may also be replaced by a different structure which is nonionic and non-chiral. In addition, for an oligonucleotide analog, a species including purine and pyrimidine other than those ordinarily found in nature may be used. As long as the oligonucleotides described above perform functions similar to those of the antisense DNA of this invention, they are encompassed by this invention as derivatives of the DNA.

In this invention, the targeted portion of mRNA to which the oligonucleotide hybridizes is preferably any of the transcription initiation site, the translation initiation site, the intron/exon binding site and the 5'-capping site. However, the site that is free from steric hindrance should be selected in consideration of the secondary structure of mRNA.

The antisense DNAs of this invention can be produced according to the methods known to one skilled in the art, e.g., the solid phase synthesis employing a DNA synthesizer available from Applied Biosystem Inc. In a similar manner its derivatives (i.e., other oligonucleotide analogs), including phosphothiate or alkyl derivatives can be produced.

### (Production of ELP and Utility Thereof)

The DNA set. forth in SEQ ID NO:2 and an expression vector obtained by introducing to a vector, sequences involved in transformation (such as a suitable promoter, origin of replication, selectable marker, RNA splicing site, or polyadenylation signal) are used to transform prokaryotic or eukaryotic host cells; the ELP of this invention can be produced by allowing the ELP gene to express in the respective host cells. Gene encoding a different protein may be linked to the DNA of this invention and a fused protein may be expressed to facilitate the purification of ELP or to increase the level of ELP expression. The ELP being produced can be sliced out by performing suitable treatment during the purification process to effect its production.

Among the hosts to be used in the expression systems for the above-stated purpose, the prokaryotic host cells include *E. coli*. and *B. subtilis*, for example. Among eukaryotes, the eukaryotic host cells include *S*. *cerevisiae* and slime moulds. Alternatively, insect cells such as Sf9 may be used as the host cell. In addition, the host cells derived from animals include COS cells and CHO cells, for example.

The ELPs of this invention produced by the techniques described above can be separated from the inside of the host cell or extracellularly and purified. For the separation and purification of ELP, there can be used separation and purification methods that are intended for ordinary proteins. For example, various kinds of chromatography, ultrafiltration, salting out, dialysis and the like may be appropriately selected and combined for use.

The ELPs of this invention thus obtained can be produced in large quantities by culturing and can be used for medical uses: specifically, therapeutic agents for the treatment of glycoprotein aberrant disorders involving calnexin and calreticulin, such as cystic fibrosis, juvenile pulmonary emphysema, Tay-Sacks disease, congenital sucrase isomaltase deficiency, and familial hypercholesterolaemia.

When the ELP of this invention is to be used in treatment, it may be administered to a patient in need of treatment by intravenous administration, local administration to the lesion, or oral administration. For purposes of administration, pharmaceutically acceptable additives (such as a carrier, an excipient, a stabilizer, a solublizing adjuvant or the like) may be added to the ELP if required, which will produce formulations adapted to the administration described above. Further, the ELP of this invention may be contained in the formulation in the form of a pharmaceutically acceptable salt. Such pharmaceutically acceptable salts include those formed with the free amino groups of protein such as those derived from hydrochloric acid, phosphoric acid, acetic acid, oxalic acid and tartaric acid and those formed with the free carboxylic acids of protein such as those derived from sodium, potassium, ammonium, calcium, ferric hydroxide, isopropylamine, triethylamine, 2-ethylaminoethanol, histidine, and procaine.

It is possible to prepare antibodies capable of recognizing an oligonucleotide having at least five consecutive amino acids within the amino acid sequence of the ELP of this invention (SEQ ID NO:1). Specifically, animals may be immunized with the oligonucletide as the antigen and the antibodies produced in their bodies may be collected and purified. Antibodies may be either polyclonal antibodies or monoclonal antibodies; the respective preparation methods are known to one skilled in the art. Either of the thus obtained anti-ELP antibodies may be used in the detection and quantification of ELP employing various kinds of immunological measurements such as enzyme immunoassays like ELISA, radioimmunoassay, and immunofluorescence technique, or may be used in the purification through the above-mentioned ELP column.

### EXAMPLES

This invention will be described more concretely by way of examples; however, the invention is not to be limited by these examples.

### (EXAMPLE. 1) Cloning Genes

The respective 24 base pairs at the 5'- and the 3'-ends of calnexin (5'-catgatggacatgatgtgacatg-3' and 5'-ctctcttcgtggctttctgtttct) were used as primers and cDNA derived from human cancer cell line A549 was used as a template to perform PCR. The PCR product was electrophoresed to determine its size and then it was subjected to cloning with a cloning kit (Original TA Cloning Kit, Invitron Inc.). The nucleotide sequence of the resulting DNA was determined using a base sequencing kit (Dye terminator Cycle Sequencing FS Ready Kit, Perkin Elmer Inc.). The thus determined sequence is shown in SEQ ID NO:2.

### (EXAMPLE 2) Isolation, Purification, and Identification of the ELP Protein

According to the conventional method, cell lysate was extracted from the human cancer cell line A549 used as the source of cDNA library in the cDNA cloning described above. After being crudely purified by gel filtration, this was further purified by electrophoresis on 7.5% SDS-PAGE. Subsequently, it was transferred to a PVDF filter and ELP blotted on the filter was subjected to a gas-phase protein sequencer, when its amino acid sequence was determined. The thus determined sequence is shown in SEQ ID NO:1.

### (EXAMPLE 3) Transfer of the ELP Gene into CF Cell

### 1. Culturing CF cell line

Human tracheal epithelial cells (CF cell line CFTE29o-) expressing Δ F508CFTR were suspended in a cell growth medium supplemented with 10% fetal bovine serum (HyClone, Lot. No. AGB 6235), antibiotic (penicillin G (100 units/ml), streptomycin (100 µ g/ml) (to a base medium (MEM(GIBCO Inc.), pH=7.4) until the level of 1.0x10⁵ cells/ml was reached. The suspension was fractionally poured into a 35-mm dish (Falcon Inc.) coated with fibronection (Nacalai Tesque. Inc.) and then stationary culturing was carried out under the conditions of 5% CO₂ and 37 °C.

### 2. Gene transfer

First, an expression vector with ELP cDNA incorporated (pCB6), 2 µg, and a plasmid for fluorescent labeling (pE-GFP-N1, CLONTECH Inc.), 2 µg, were precipitated with EtOH and dissolved in a serum-free medium (MEM), 30 µl (Solution A). Transfectam reagent (Promega Inc.), 10 µl, was next added to a serum-free medium, 300 µl (Solution B). Solutions A and B were mixed and allowed to react at room temperature for 10 minutes (Solution C). This Solution C was added to the subconfluent CF cells (CFTE29o⁻, 70%) that had been grown according to the method described in 1 above and then culturing was carried out for 2 hours under the conditions of 5% CO₂ and 37 ° c. After culturing, the resulting solution was removed and a medium containing serum was added thereto; and culturing was carried out for 2 days under the conditions of 5% CO₂ and 37 ° c.

Two days after gene transfer the cells were twice washed with an extracellular fluid (150 mM NaCl, 2.5 mM CaCl₂, 2.5 mM MgCl₂, 10 mM HEPES, with pH: 7.3 adjusted by NaOH) followed by the addition of the extracellular fluid (1ml). The petri dish was then placed on a fluorescence microscope (IMT-20, BH2-RFL-T3, Olympus Optical Co. Ltd.) and irradiated with a 488-nm excitation light, under which the cells were observed. Only the cells emitting green fluorescence (expression of GFP marker) were examined in the patch clamp method described below.

### (EXAMPLE 4) The Patch Clamp Method (Cell-Attached Mode)

The patch clamp method as described in FEBS Letters, 455, 215-218, 1999 was performed to determine the effect of ELP on the glycoprotein (CFTR), employing 16HBE14o⁻ cells (expressing the normal CFTR), CFTE29o⁻ cells (transfected with the ELP gene), and CFTE29o⁻ cells as the control (to which only the vector pCB6 was introduced) or CFTE29o⁻ cells with no gene transfer as the control.

The electrodes (patch electrodes) for use in the patch clamp method (GC120TF-10, Clark Electromedical Instruments Inc.) had been fabricated with a minute electrode-assembling device (P-97, Sutter Instruments Inc.) and heat-polished with a microforge (MF-830, NARISIGE Co. Ltd.), which were employed. This patch electrode was brought into light contact with the cell surfaces by a three dimensional water pressure micromanipulator (MV-3, NARISIGE Co. Ltd.) while square wave voltage pulses were being applied to the electrode; by lightly applying voltage to the electrode, gigaohm seal (GΩseal) was formed. A patch clamp amplifier (AXOPATCH 1D, Axon Instruments Inc.) was used to record current and to maintain membrane potential. The obtained waveform was observed and analyzed with an oscilloscope (DSO450, GOULD Inc.).

In 16HBE14o⁻ cells expressing the normal CFTR, outward CFTR current (ca. 0.4 pA) was observed at a holding potential of 40 mV when forskolin, 10 µM, was administered to the cell, whereas in CFTE29o- cells expressing Δ 508CFTR no CFTR current was observed. In the CFTE29o⁻ cells to which the ELP gene had been introduced, the outward CFTR current was observed after administration. When the vector (pCB6) was introduced to the cell (which served as the control), no CFTR current was observed.

These experimental results confirmed that the ELP enhanced the expression of Δ F508CFTR on the cell membrane and the expressed Δ F508CFTR had its normal function.

### Industrial Applicability

Calnexin and calreticulin display the function of the control of folding and assembling membrane-binding glycoproteins such as CFTR as well as the function of suppressing the expression and secretion of those proteins in the event of their aberration. Since the ELP of this invention controls . the function of calnexin, it inhibits mutant glycoprpteins which do not dissociate from calnexin from remaining in the ER for a prolonged period of time; therefore, it can be used in the treatment of glycoprotein aberration disorders (e.g., cystic fibrosis) resulting from the retention.

The DNA of this invention also encodes the ELP protein; therefore, when it is applied in the gene therapy, the DNA produces the ELP intracellularly and is useful in the treatment of the glycoprotein aberration disorders.

## Claims

1. A protein comprising the amino acid sequence set forth in SEQ ID NO:1 in the Sequence Listing.

2. A protein comprising an amino acid sequence derivable from the substitution or the deletion of one or more amino acids in the amino acid sequence SEQ ID NO:1 in the Sequence Listing, or from the addition of one or more amino acids to the amino acid sequence set forth in SEQ ID NO:1 in the Sequence Listing, the protein possessing glycoprotein production regulating

3. DNA comprising the nucleotide sequence set forth in SEQ NO:2 in the Sequence Listing.

4. DNA comprising a nucleotide sequence derivable from the substitution or the deletion of one or more nucleotides in the nucleotide sequence set forth in SEQ ID NO:2 in the Sequence Listing, or from the addition of one or more nucleotides to the nucleotide sequence set forth in SEQ ID NO:2 in the Sequence Listing.

5. Antisense DNA to the DNA according to claims 3 or 4.

6. A therapeutic agent for the treatment of a glycoprotein aberration disorders, comprising the protein according to claims 1 or 2.

7. A gene therapy agent for the treatment of a glycoprotein aberration disorder, comprising the DNA according to claims 3 or 4.


SEQUENCE LISTING

<110> Hisamitsu Pharmaceutical Co., Inc.

<120> Novel endoplasmic localization proteins, therapeutic agents for gl
ycoprotein aberration disorders containing same, DNA encoding the protei
ns, and therapeutic agents in gene therapy for glycoprotein aberration d
isorders

<130> FP00-0271-00

<150> JP 11/344795
<151> 1999-12-03

<160> 2

<170> PatentIn Ver. 2.0

<210> 1
<211> 256
<212> PRT
<213> Human

<400> 1
Met Glu Gly Lys Trp Leu Leu Cys Met Leu Leu Val Leu Gly Thr Ala
1               5                   10                  15
Ile Val Glu Ala His Gly Gly His Asp Asp Asp Met Ile Asp Ile Glu
            20                  25                  30
Asp Asp Leu Asp Asp Val Ile Glu Glu Val Glu Asp Ser Lys Ser Lys
        35                  40                  45
Ser Asp Ser Ser Thr Pro Pro Ser Pro Lys Val Thr Tyr Lys Ala Pro
    50                  55                  60
Val Pro Thr Gly Gly Val Tyr Phe Ala Asp Ser Phe Asp Arg Gly Ser
65                  70                  75                  80
Leu Ser Gly Trp Ile Leu Ser Lys Ala Lys Lys Asp Asp Thr Asp Asp
                85                  90                  95

Glu Ile Ala Lys Tyr Asp Gly Lys Trp Glu Val Asp Glu Met Lys Asp
            100                 105                 110
Thr Lys Leu Pro Gly Asp Lys Gly Leu Val Leu Met Ser Arg Ala Lys
        115                 120                 125
His His Ala Ile Ser Ala Lys Leu Asn Lys Pro Phe Leu Phe Asp Thr
    130                 135                 140
Lys Pro Leu Ile Val Gln Tyr Glu Val Asn Phe Gln Asn Gly Ile Glu
145                 150                 155                 160
Cys Gly Gly Ala Tyr Val Lys Leu Leu Ser Lys Thr Ser Glu Leu Asn
                165                 170                 175
Leu Asp Gln Phe His Asp Lys Thr Pro Gln Pro Asp Val Lys Glu Glu
            180                 185                 190
Asp Gly Lys Glu Glu Glu Lys Asn Lys Gly Asp Glu Glu Glu Glu Gly
        195                 200                 205
Glu Glu Lys Leu Glu Glu Lys Gln Lys Ser Asp Ala Glu Asp Asp Gly
    210                 215                 220
Gly Thr Gly Ser Gln Asp Glu Glu Asp Arg Lys Pro Thr Ala Glu Glu
225                 230                 235                 240
Asp Glu Ile Leu Asn Arg Ser Pro Arg Asn Arg Lys Pro Arg Arg Glu
                245                 250                 255

<210> 2
<211> 771
<212> DNA
<213> Human

<400> 2
atggaaggga agtggttgct gtgtatgtta ctggtgcttg gaactgctat tgttgaggct 60
catggtggac atgatgatga catgattgat attgaagatg atcttgatga tgttattgaa 120
gaggtagaag attcgaaatc gaaatcagat tccagtactc ctccatctcc aaaggtcacc 180
tacaaagctc cagttccaac agggggagtt tattttgctg actcctttga tagagggtct 240
ctatcagggt ggattttatc taaagccaaa aaagatgaca ctgatgatga aattgccaaa 300
tatgatggaa agtgggaggt agatgaaatg aaggacacaa agcttccagg tgataaagga 360
cttgtactga tgtctcgagc caagcatcat gccatctctg ctaaactgaa caagcccttc 420
ctgtttgaca ccaagcctct catcgttcag tatgaggtta attttcagaa tggaatagaa 480
tgtggtggtg cctatgtgaa gctgctttcc aagacctcag aactcaactt ggatcaattc 540
cacgacaaga ctccccagcc agatgtgaag gaagaagatg ggaaggaaga agagaagaac 600

aagggggatg aagaggaaga aggagaagag aagcttgaag agaaacagaa aagtgatgct 660
gaagatgatg gtggcactgg cagtcaagat gaggaagata gaaaacctac tgcagaggag 720
gatgaaatct tgaacagatc accaagaaac agaaagccac gaagagagtg a 771